# EUROPEAN PATENT SPECIFICATION

(11) **EP 2 240 233 B1**
(45) Date of publication and mention of the grant of the patent: **22.08.2012**
(21) Application number: 08864250.9
(22) Date of filing: 18.12.2008
(51) Int. Cl.: A61M 39/00, A61M 39/10, A61M 39/28

(54) **CONNECTORS**
STECKVERBINDER
RACCORDS

(30) Priority: 20.12.2007 GB 0724839
(43) Date of publication of application: 20.10.2010
(73) Proprietor: Smith&Nephew PLC, London WC2N 6LA (GB)
(72) Inventor: HALL, Kristian, Hull HU13 0HN (GB); HARTWELL, Edward, York YO10 5DF (GB); RICHARDSON, Martin, Grimsby DN37 9RE (GB)
(74) Representative: Uno, Jennifer Elizabeth Hayes
(86) International application number: PCT/GB2008/051202
(87) International publication number: WO 2009/081196

(56) References cited:
- EP-A- 0 340 427
- US-A- 4 187 846
- US-A- 4 473 369
- US-A1- 2006 129 110

## Description

The present invention relates to connectors for joining or separating a plurality of conduits particularly, though not exclusively, in the field of medical devices for establishing or stopping fluid flow through the conduits.

Many medical devices require the provision of conduits, particularly flexible conduits made from relatively soft plastics material tubing, for conveying various fluids to and from a patient. It is frequently necessary to interrupt the flow of fluid between a device and a patient in order to, for example, replace a source of fluid or to replace a receptacle receiving fluid from a patient, for example. Examples of devices which requires fluid flow conduits to be provided between patient and device are topical negative pressure (TNP) therapy devices which are well known in the medical device art.

Frequently, conduits are merely pushed onto or pulled off a tubular spigot associated with the device to effect replacement of the device or item in question. In other instances conduits may be cut and clamps of various types used to seal off the open ends of the severed conduit.

It is a fact that patients, especially in hospitals for example, may have many conduits attached to them and it is important that connectors in conduits used to link medical devices to patients should be as foolproof as possible and that the connectors should have unique features so that they cannot be misconnected to a patient. Similarly, a device may have a plurality of conduits conveying different fluids to and from a patient and it is important that such conduits cannot be crossed over or be wrongly connected. Furthermore, the ability to ensure correct connection of various fluids to required sites between patient and devices also helps to minimise the spread of biohazard and cross contamination.

In our co-pending International patent application, WO 2004/037334, apparatus, a wound dressing and a method for aspirating, irrigating and cleansing wounds are described. In very general terms, this invention describes the treatment of a wound by the application of topical negative pressure (TNP) therapy for aspirating the wound together with the further provision of additional fluid for irrigating and/or cleansing the wound, which fluid, comprising both wound exudates and irrigation fluid, is then drawn off by the aspiration means and circulated through means for separating the beneficial materials therein from deleterious materials. The materials which are beneficial to wound healing are recirculated through the wound dressing and those materials deleterious to wound healing are discarded to a waste collection bag or vessel.

In our co-pending International patent application, WO 2005/04670, apparatus, a wound dressing and a method for cleansing a wound using aspiration, irrigation and cleansing wounds are described. Again, in very general terms, the invention described in this document utilises similar apparatus to that in WO 2004/037334 with regard to the aspiration, irrigation and cleansing of the wound, however, it further includes the important additional step of providing heating means to control the temperature of that beneficial material being returned to the wound site/dressing so that it is at an optimum temperature, for example, to have the most efficacious therapeutic effect on the wound.

In our co-pending International patent application, WO 2005/105180, apparatus and a method for the aspiration, irrigation and/or cleansing of wounds are described. Again, in very general terms, this document describes similar apparatus to the two previously mentioned documents hereinabove but with the additional step of providing means for the supply and application of physiologically active agents to the wound site/dressing to promote wound healing.

The content of the above references is included herein by reference.

In the above referenced International patent applications there may be in one TNP apparatus several conduits such as:
1) a conduit to irrigate a wound, i.e. put liquid into a wound and pass back to a device or waste container;
2) a conduit to apply a vacuum to a wound cavity
3) a conduit to measure pressure in the wound cavity and/or to provide an air bleed into a wound cavity; and
4) a conduit to inflate/deflate a wound filling bag device.

Furthermore, it is entirely possible that additional conduits between a patient and apparatus may also need to be accommodated.

Thus, it may be seen that it is a necessity that connectors are as foolproof as possible especially when it is desired to connect/disconnect all conduits simultaneously.

It is also desirable that a connector which connects four separate conduits, for example, also prevents flow through the conduits on both sides of the connector when a conduit connector is separated into its two connecting portions.

In apparatus where there is an irrigant conduit it is critical to seal this line securely as it is frequently pressurised by pumping or, at times, may be under negative pressure, for example, at start up and as the fluid is entering the wound it can carry bioburden direct to the wound.

EP0340427 relates to a catheter connector having a single clamp for a catheter tube that may have multiple lumens.

It is an object of the present invention to provide a connector for connecting a plurality of conduits and to be able to connect and separate those conduits simultaneously and, further to be able to stem the flow of fluids in both parts of the separated conduits when the connector is separated into its constituent halves.

The invention is defined in the appended claims.

According to the present invention there is provided a connector for the connection of a plurality of conduits, the connector comprising: a male connector portion and a female connector portion; the male connector portion having a plurality of male conduit connection spigot members sealingly engageable with a plurality of female conduit connection socket members in the female connector portion; the spigot members and socket members having conduit receiving means; a conduit clamp member in each of the male and female connector portions; mutual engagement means in the male and female connector portions for holding the connector portions together or for allowing separation of the connector portions, the engagement means being responsive to fluid flow stemming and fluid flow permitting positions of the clamp members.

The connector according to the present invention may have provision to accommodate a plurality of fluid flow conduits such as, for example, four conduits connected to the male connector portion and to the female connector portion. However, even though the connector according to the present invention may have provision to accommodate four conduits, the connector may be used utilising a lesser number of conduits.

The conduits may or may not be all of the same diameter or size of outer diameter or inner diameter bore. The male connection spigots and female connection sockets may be sized suitably so as to utilise a size of conduit appropriate to a flow rate, for example, required in that particular conduit.

The conduits may be comprised of so-called "Para" tubing where a plurality of separate lumens are provided in a single conduit body with the separate lumens bonded to a neighbouring lumen and which lumens may be peeled apart as required to fit to the connector portions and to apparatus fluid connections. Again, it is not necessary that all lumens be of the same size.

The conduits may be made from soft, flexible plastics materials such as PVC, polyurethane, silicone, polyethylene and polypropylene, for example.

The conduit receiving means on the male spigots and female sockets may be pockets within which ends of the conduits are received or may, for example, be tubular spigots onto which conduits may be pushed. However, in the latter case, barbed spigots increase the size of the connector.

The male connection spigot members and female socket members sealingly engage one another. Sealing engagement may be effected by means of seal members on one of the engaging members, for example. Such seal members may comprise "O" rings, for example, on the male spigot member, for example.

The male spigot members and the female socket members may have, in one embodiment of the present invention, pockets to receive conduits therein. The conduits may be held in the pockets by friction, adhesive means or be welded therein, for example, or any other suitable means of ensuring retention of the conduit in a pocket.

The male spigot members and the female socket members may be held and located in their respective connector portions by mutual engaging features on the spigots and sockets with locating features provided in body portions of the male and female connector portions so as to locate the spigot and socket members in predetermined positions to permit easy engagement of the male and female connector portions when aligned.

In one embodiment of the connector according to the present invention, the male spigot members and/or the female socket members may be provided on a manifold member comprising the plurality of spigots and/or sockets.

A conduit clamp member is provided in each connector half. In one embodiment of the present invention the conduit clamp member may be provided with a plurality of conduit closing means corresponding to the number of conduits which may be accommodated by the connector. The conduit closing means may, for example, comprise apertures through which the conduits pass in fluid flow permitting manner and slot portions sited on peripheries of the apertures, the slot portions being substantially parallel to each other and, when the conduits are in the slot portions they are squeezed so that fluid flow is stemmed. The clamp member may be arranged in each connector portion to be moveable transversely to the conduits so that the conduits may be brought into and out of engagement with the slot portions

The connector portions may have mutual engagement means which are responsive to the position of the conduit clamp members. In this regard the male and female connector portions may be provided with mutual engagement means which either hold the connector portions together in secure flow permitting engagement or, allow the connector portions to be pulled apart in a fluid flow stemming condition. The conduit clamp member has two positions: a first position where the conduits are located in apertures, for example, as described above and in which position fluid flow is permitted and the mutual engagement means prevent the male and female connector portions from being separated; and, a second position wherein the conduits are located in the slot portions, the conduits are squeezed closed and the mutual engagement means become disengagable and separation of the male and female connector portions may be easily achieved.

The male and female connector portions may have body portions of "shell-like" construction in which all of the components of the connector portions may be contained. The body portions may have suitable mutual engagement means as described above and which mutual engagement means may comprise finger and slot snap-fit engagement features or finger and slot features which are biased into engagement with each other depending upon the position of the conduit clamp member, for example.

Desirably the male and female connector portions of the connector according to the present invention may be "handed" in that they cannot be connected together the wrong way around.

Although the two connector portions are held in firm engagement by the engagement means when the conduit clamp member is in a fluid flow permitting position, should the conduits become entangled with a patient, for example, and a stress is placed on the connector it may become disengaged at an axial force applied via the conduits of in the region of 10 to 80N preferably 20 - 40N on the connector.

In order that the present invention may be more fully understood and example will now be described by way of illustration only with reference to the accompanying drawings, of which:
Figure 1 shows a cross section through a male connector portion;
Figure 2 shows a cross section through a female connector portion;
Figure 3 shows a view in side elevation of a connector according to the present invention wherein the male and female connector portions are separated;
Figures 4A to 4E show various views of a conduit clamp member, Figure 4A showing a front view, Figure 4B showing a side view, Figure 4C showing a view 180º to that of Fig. 4A, Figure 4D showing a perspective view of a conduit clamp member from a three-quarter front view, and Figure 4E which shows a cross section on the line 4E-4E of Figure 4A;
Figure 5 shows a cross section through a manifold having a plurality of female conduit connection socket members;
Figures 6A to 6C show various views of a connector portion cap to protect fluid connections when connector portions have been separated;
Figure 7 shows a cross section on the line 7-7 of Figure 1; and
Figure 8 which shows across section on the line 8-8 of Figure 3.

Referring now to the drawings and where the same features are denoted by common reference numerals.

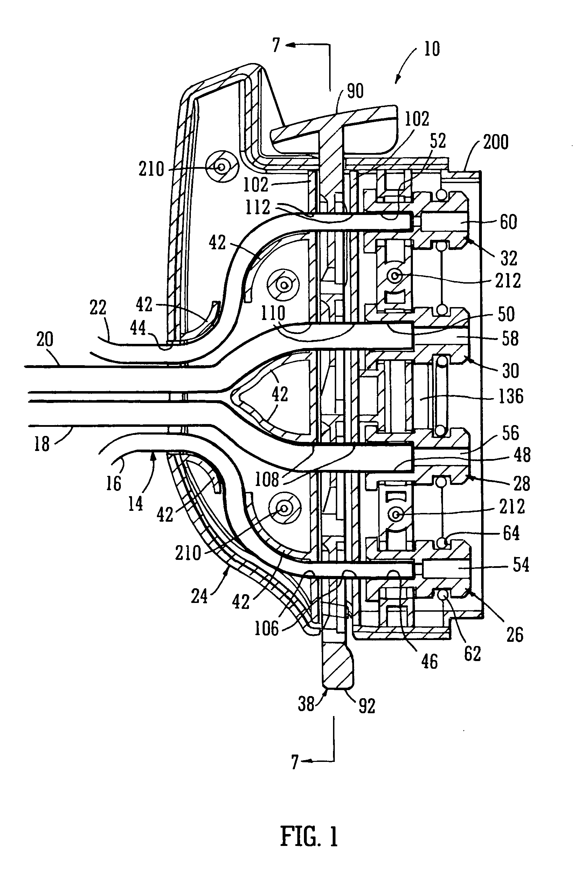

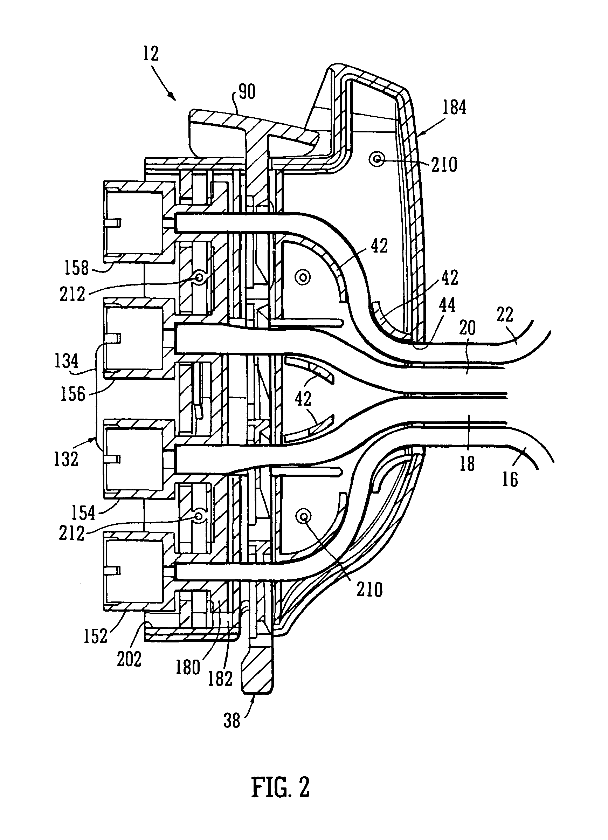

In Figures 1 and 2 cross sections of male and female connector portions 10, 12 are shown, respectively. The male connector portion 10 of Figure 1 and the female connector portion 12 of Figure 2 show a four-lumen conduit 14 having lumens 16, 18, 20 and 22; centre lumens 18 and 20 being of larger diameter than outer lumens 16 and 22. The lumen reference numerals are maintained in each connector portion to indicate that they form a fluid flow through passage in the connected portions. The conduit 14 is so-called "Para" tubing and all four lumens are bonded together but easily separated by peeling apart where required.

In the detailed description below common reference numerals are used to denote features of construction and the like in the male 10 and female 12 connector portions where those features have the same function and/or effect in each connector portion.

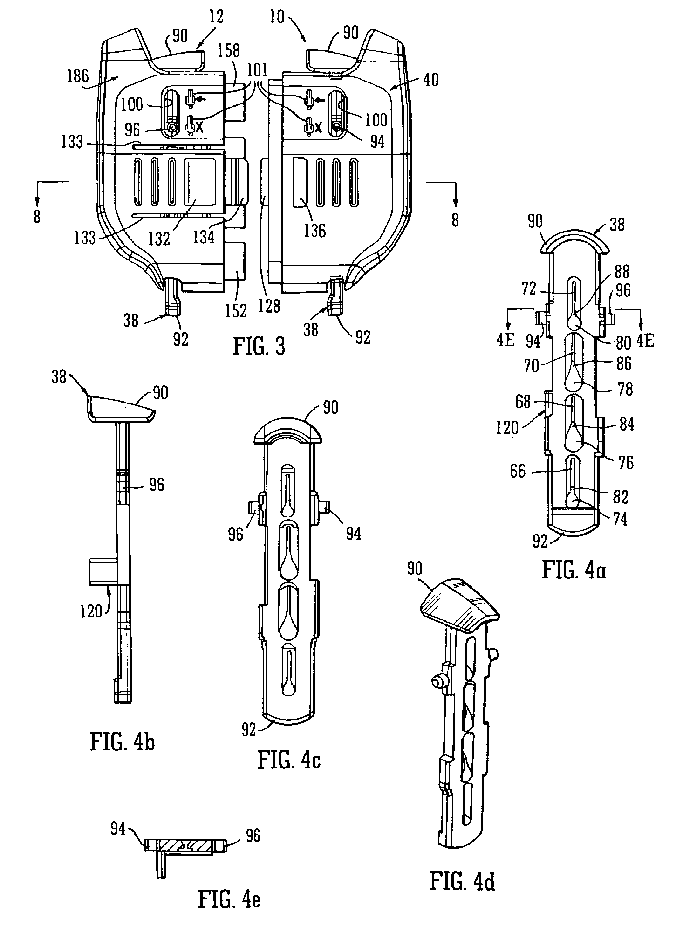

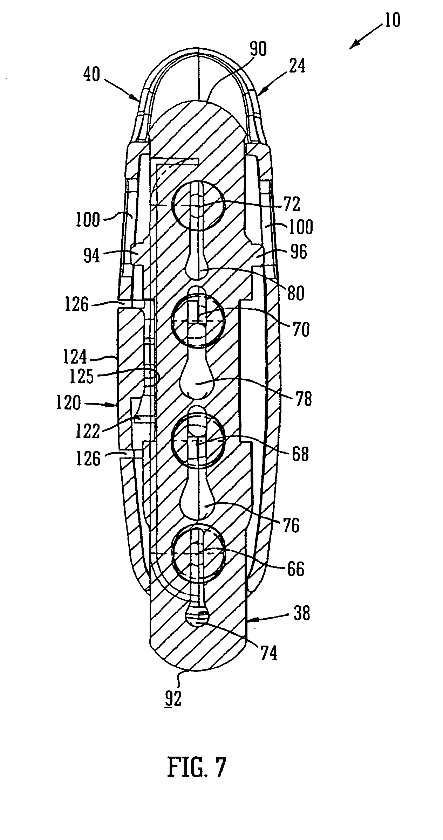

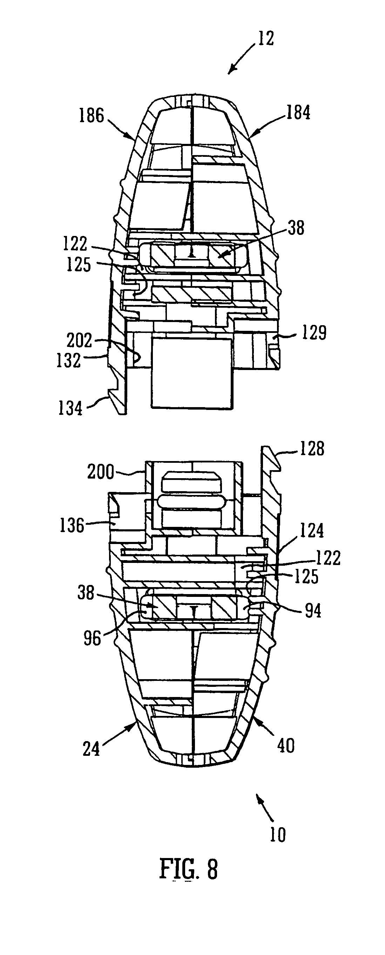

The male connector portion 10 comprises: a body portion 24; four male conduit connection spigots 26, 28, 30, 34 which connect with lumens 16, 18, 20, 22, respectively; and, a conduit clamping member 38. The body portion 24 shown in Figure 1 has a substantially mirror image body portion 40 (best seen in Figure 3 which shows the connector portions 10, 12 from the reverse side) except for important differences which will be explained in more detail below. The body portions 24, 40 have conduit guide walls 42 which prevent the lumens 16, 18, 20, 22 from becoming kinked as the conduit 14 enters a conduit entry aperture 44 which is suitably dimensioned so as to lightly grip the four lumens and prevent then from being easily pulled out of the connector portion 10. The lumens 16, 18, 20, 22 are received in appropriately sized pockets 46, 48, 50, 52, respectively in the male spigots 26, 28, 30, 32 and are retained therein by an adhesive. The male spigots have through passages 54, 56, 58, 60. Each of the male spigots is provided with an "O" ring seal 62 in a groove 64 to seal with co-operating female socket members to be described below. The conduit clamp member 38 is shown in the conduit closing, fluid flow stemming position where the conduits 16, 18, 20, 22 are squeezed in slots 66, 68, 70, 72 to prevent fluid flow through all conduits simultaneously (the conduit clamping member 38 is best seen in Figure 4). The slide clamp 38 (and the corresponding slide clamp in the female connector portion 12 which is identical) has four apertures 74, 76, 78, 80 in which the four conduits 16, 18, 20, 22 are located, respectively when the slide clamp member 38 is in the open fluid flow permitting position (to be explained below), the slots 66, 68, 70, 72 leading off from the apertures via tapered portions 82, 84, 86, 88 on the aperture peripheries and leading into the slots 66, 68, 70, 72. The conduit clamping member 38 has thumb tab 90 to aid pushing down into the fluid flow stemming position (as shown in Figs 1 and 2) and serrated gripping surface 92 at its distal end to aid pushing the clamping member into its fluid flow permitting position. The conduit clamping member 38 has two guide spigots 94, 96 which run in slots 98, 100 in body portions 24, 40, respectively. The guide spigots 94, 96 also provide an additional indication as to whether the conduit slide clamping member 38 is in a flow stemming or flow permitting position by icons 101 moulded on the body portion 24, 40. The slide clamping members 38 run in guide plates 102 which also possess recesses 106, 108, 110, 112 to locate the conduits 16, 18, 20, 22 in the direction of movement of the clamping member so that the conduits can be closed off and opened without them being physically shifted by movement of the clamping member. The conduit clamping member 38 also has a cam arrangement 120 associated with engagement means for locking and unlocking the male and female connector portions to and from each other depending upon the position of the conduit slide clamping member 38 (the description of the engaging arrangement is best seen with reference to Figures 3, 4, 7 and 8). The cam arrangement 120 comprises a cam portion 122 which acts upon a wall portion 124 having a camming surface 125, the wall portion 124 being separated from the body portion 24 by two slits 126 parallel to the direction of engagement of the male and female connector portions 10, 12. When the conduit slide clamping member 38 is in the fluid flow stemming position as shown in the drawings with the conduits squeezed off to prevent fluid flow, the cam portion 122 does not engage the camming surface 125. However, when the slide clamp 38 is moved to the flow permitting position the cam portion 122 moves upwardly relative to the camming surface 125 as shown in Figure 7 and pushes the wall portion 124 in an outwardly direction. This has the effect of moving an engaging finger 128 on the lower extremity of the wall portion 124 outwardly to engage a slot 129 in the body portion 184 of the female connector portion 12. Movement of the conduit slide clamp member 38 in the female connector portion 12 to the flow permitting position has precisely the same effect in moving a flexible wall portion 132 defined by slits 133 on the body portion 186 on the female connector portion 12, the wall portion 132 having a corresponding finger 134 which is moved into firm engagement with a corresponding slot 136 on the body portion 24 of the male connector portion 10.

The female connector portion 12 has most of the same features as the male connector portion 10 and these are given the same reference numerals where these features operate in an identical manner to those features of the male connector portion 10.

One area where the female connector portion 12 differs from the male connector portion 10 is in the actual fluid connection means allowing the male and female connector portions to sealingly connect with each other to permit fluid flow. The female connector portion 12 has a manifold member 150 having thereon four female conduit connection socket members 152, 154, 156, 158 (shown clearly in Fig. 4) which sealingly engage with the male conduit connection spigot members 26, 28, 30, 32. The "O" ring seals 64 on the spigot members seal with bores 160, 162, 164, 166 in the female socket members. The female socket members have through passages 168, 170, 172, 174 which are in fluid communication with the conduits 16, 18, 20, 22. The conduits are received in pockets in the female socket members in the same manner as with the male spigot members and will not be described further. The female socket members are moulded integrally with a common base plate 180 which is located in a channel 182 in body portion parts 184, 186 which, as with body portion parts 24, 40 of the male connector portion 10, are essentially mirror images of each other apart from some differences as explained below. The co-operating male spigot members 26, 28, 30, 32 have a small degree of movement because they are individual items held in the body portions 24, 40 and may move slightly to align themselves with the bores160, 162, 164, 166 of the manifold member 150 which holds the female socket members in relatively rigid array.

The male connector portion 10 has a skirt portion 200 about its lower periphery and which skirt portion 200 fits inside a co-operating skirt portion 202 on the female connector portion 12 to aid alignment when joining the two connector portions together and also to limit the ingress of dirt and contaminants to the region in which the conduit connections are made.

The two body portions 24, 40 of the male connector portion 10 and the two body portions 184, 186 of the female connector portion 12 have co-operating bosses 210, 212 to allow the body half portions 24, 40 and 184, 186 to be joined together. The bosses 210, 212 include spacers so that the body portion halves are accurately located relative to each other to allow free sliding movement of the conduit slide clamp 38 without binding.

The body portions 24, 186 are identical to each other as are body portions 40, 184. Thus, the connector is made more economic by reducing the number of mouldings required. However, due to the engagement arrangement of co-operating fingers and slots being "handed", it is not possible to connect the male and female connector portions the wrong way around. The conduit slide clamp members are also identical to each other in each connector portion.

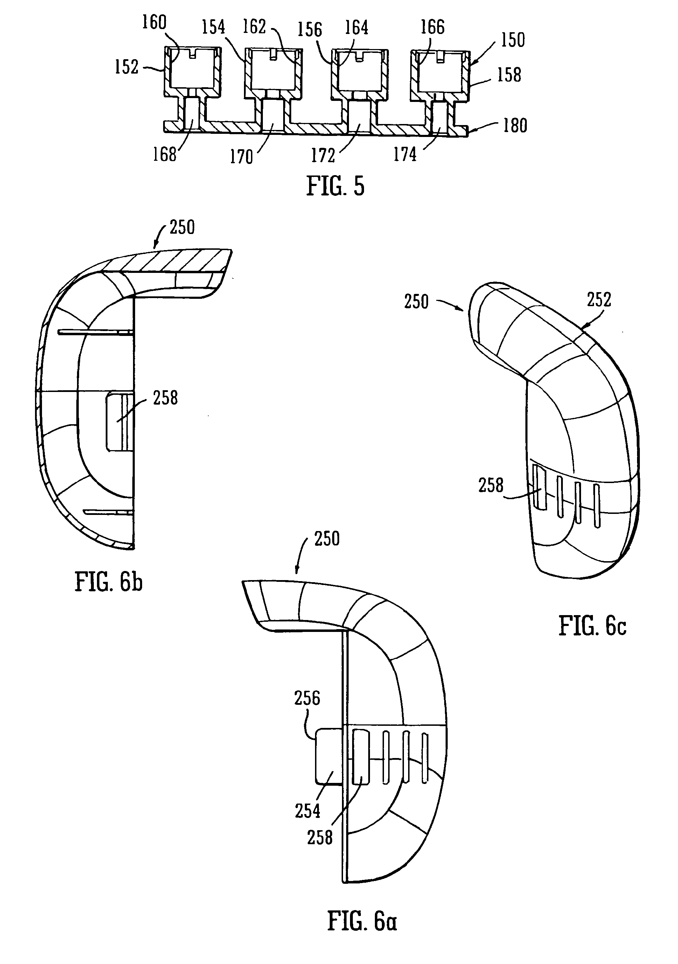

A cap member 250, shown in Figure 6, is also provided to prevent the ingress of dirt and contamination to the conduit connection spigots and socket regions of each connector portion when separated. The cap member 250 has a contoured shell portion 252 to cover the region of spigot and socket conduit connection members when the connector is separated. The cap member is held onto a connector portion by means of a flexible tongue 254 having a finger 256 (analogous to fingers 128, 134) which engage with slot 136 or that of the female connector portion 12 as appropriate and the finger portion 128, 134 of the connector portion engages with a slot 258 in the cap member as appropriate. The cap members are identical for both connector portions.

Throughout the description and claims of this specification, the words "comprise" and "contain" and variations of the words, for example "comprising" and "comprises", means "including but not limited to", and is not intended to (and does not) exclude other moieties, additives, components, integers or steps.

Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith.

## Claims

1. A connector for the connection of a plurality of conduits, the connector comprising: a male connector portion (10) and a female connector portion (12); the male connector portion having a plurality of male conduit connection spigot members (26, 28, 30, 34) sealingly engageable with a plurality of female conduit connection socket members (152, 154, 156, 158) in the female connector portion; the spigot members and socket members having conduit receiving means (46, 48, 50, 52); mutual engagement means (120) in the connector portions for holding the male and female connector portions together or for allowing separation of the connector portions, **characterised in that** the connector further comprises a conduit clamp member (38) in each of the male and female connector portions; the engagement means being responsive to fluid flow stemming and fluid flow permitting positions of the clamp members (38).

2. A connector according to claim1 wherein the conduit receiving means are selected from one of: pockets for receiving a conduit therein; and tubular spigots for receiving a conduit thereon.

3. A connector according to claim 1 or claim 2 wherein the conduits have different sizes.

4. A connector according to any one preceding claim wherein the male spigots and female sockets have sealing means (62, 64) therebetween.

5. A connector according to any one preceding claim wherein the male spigot members or the female socket members are provided on a manifold member (150).

6. A connector according to claim 5 wherein the other of the male spigot members or female socket members are individually located within the connector portion.

7. A connector according to any one preceding claim wherein the conduit clamp member (38) has apertures (74, 76, 78, 80) through which conduits pass in a fluid flow condition and slot portions (66, 68, 70, 72) in which conduits are squeezed closed in a non-fluid flow condition.

8. A connector according to claim 7 wherein in the conduit clamp member (38) the slot portions (66, 68, 70, 72) are connected to the apertures at a periphery thereof.

9. A connector according to claim 7 or claim 8 wherein the conduit clamp member (38) is moveable in a direction substantially parallel to said slot portions.

10. A connector according to any one of preceding claims 7 to 9 wherein all conduits are opened or closed simultaneously by movement of the conduit clamp member (38).

11. A connector according to any one preceding claim wherein the conduit clamp member (38) is moveable in a direction transverse to axes of the spigot and socket connection members.

12. A connector according to any one preceding claim wherein the engagement means (120) are held in engagement to hold the male and female connector portions together when the conduit clamp member is in a fluid flow permitting position.

13. A connector according to any one preceding claim wherein the mutual engagement means (120) are releasable when the conduit clamp members are in a fluid flow stemming position.

14. A connector according to any one preceding claim wherein the mutual engaging means (120) comprise engaging finger (128, 134) and slot portions (129, 136).

15. A connector according to claim 14 wherein the conduit clamp member (38) has camming means (122, 125) which push the finger and slot portions into engagement when the conduit clamp member is in a fluid flow permitting position.

16. A connector according to any one preceding claim wherein the male and female portions (10, 12) are handed to prevent incorrect assembly.

## Patentansprüche

1. Ein Verbindungsteil zur Verbindung einer Vielzahl von Schläuchen, wobei das Verbindungsteil Folgendes beinhaltet: einen Außenverbindungssteilabschnitt (10) und einen Innenverbindungsteilabschnitt (12); wobei der Außenverbindungssteilabschnitt eine Vielzahl von Außenschlauchverbindungszapfenelementen (26, 28, 30, 32) aufweist, die mit einer Vielzahl von Innenschlauchverbindungssteckerelementen (152, 154, 156, 158) in dem Innenverbindungsteilabschnitt abdichtend in Eingriff gebracht werden können; wobei die Zapfenelemente und die Steckerelemente Schlauchaufnahmemittel (46, 48, 50, 52) aufweisen; gegenseitige Eingriffsmittel (120) in den Verbindungsteilabschnitten zum Zusammenhalten des Außenverbindungsteilabschnitts und des Innenverbindungsteilabschnitts oder zum Erlauben der Trennung der Verbindungsteilabschnitte, **dadurch gekennzeichnet, dass** das Verbindungsteil ferner in jeweils dem Außenverbindungsteilabschnitt und dem Innenverbindungsteilabschnitt ein Schlauchklemmenelement (38) beinhaltet; wobei die Eingriffsmittel auf Fluidströmungshemm- und Fluidströmungsermöglichungspositionen der Klemmenelemente (38) reagieren.

2. Verbindungsteil gemäß Anspruch 1, wobei die Schlauchaufnahmemittel aus einem von Folgendem ausgewählt sind: Aussparungen zum Aufnehmen eines Schlauches darin; und röhrenförmigen Zapfen zum Aufnehmen eines Schlauches darauf.

3. Verbindungsteil gemäß Anspruch 1 oder Anspruch 2, wobei die Schläuche unterschiedliche Größen aufweisen.

4. Verbindungsteil gemäß einem der vorhergehenden Ansprüche, wobei die Außenzapfen und die Innenstecker Abdichtungsmittel (62, 64) dazwischen aufweisen.

5. Verbindungsteil gemäß einem der vorhergehenden Ansprüche, wobei die Außenzapfenelemente oder die Innensteckerelemente auf einem Mehrfachelement (150) bereitgestellt sind.

6. Verbindungsteil gemäß Anspruch 5, wobei das andere von den Außenzapfenelementen oder den Innensteckerelementen innerhalb des Verbindungsteilabschnitts einzeln angeordnet ist.

7. Verbindungsteil gemäß einem der vorhergehenden Ansprüche, wobei das Schlauchklemmenelement (38) Öffnungen (74, 76, 78, 80), durch die in einem Fluidströmungszustand Schläuche führen, und Schlitzabschnitte (66, 68, 70, 72), in denen in einem Fluidnichtströmungszustand Schläuche zugedrückt werden, aufweist.

8. Verbindungsteil gemäß Anspruch 7, wobei in dem Schlauchklemmenelement (38) die Schlitzabschnitte (66, 68, 70, 72) an einem Umfang davon mit den Öffnungen verbunden sind.

9. Verbindungsteil gemäß Anspruch 7 oder Anspruch 8, wobei das Schlauchklemmenelement (38) in einer Richtung, die im Wesentlichen parallel zu den Schlitzabschnitten verläuft, bewegbar ist.

10. Verbindungsteil gemäß einem der vorhergehenden Ansprüche 7 bis 9, wobei alle Schläuche durch die Bewegung des Schlauchklemmenelements (38) gleichzeitig geöffnet oder geschlossen werden.

11. Verbindungsteil gemäß einem der vorhergehenden Ansprüche, wobei das Schlauchklemmenelement (38) in einer Richtung, die zu den Achsen der Zapfen- und Steckerverbindungselementen quer verläuft, bewegbar ist.

12. Verbindungsteil gemäß einem der vorhergehenden Ansprüche, wobei die Eingriffsmittel (120) in Eingriff gehalten werden, um den Außenverbindungsteilabschnitt und den Innenverbindungsteilabschnitt zusammenzuhalten, wenn sich das Schlauchklemmenelement in einer Fluidströmungsermöglichungsposition befindet.

13. Verbindungsteil gemäß einem der vorhergehenden Ansprüche, wobei die gegenseitigen Eingriffsmittel (120) lösbar sind, wenn sich die Schlauchklemmenelemente in einer Fluidströmungshemmposition befinden.

14. Verbindungsteil gemäß einem der vorhergehenden Ansprüche, wobei die gegenseitigen Eingreifmittel (120) Eingreiffinger- (128, 134) und -schlitzabschnitte (129, 136) beinhalten.

15. Verbindungsteil gemäß Anspruch 14, wobei das Schlauchklemmenelement (38) Nockenmittel (122, 125) aufweist, die die Finger- und Schlitzabschnitte in Eingriff schieben, wenn sich das Schlauchklemmenelement in einer Fluidströmungsermöglichungsposition befindet.

16. Verbindungsteil gemäß einem der vorhergehenden Ansprüche, wobei der Außenabschnitt und der Innenabschnitt (10, 12) gebildet sind, um falsches Zusammenbauen zu verhindern.

## Revendications

1. Un raccord pour le raccordement d'une pluralité de tuyaux, le raccord comprenant : une portion de raccord mâle (10) et une portion de raccord femelle (12) ; la portion de raccord mâle possédant une pluralité d'éléments formant bouts unis de raccordement de tuyau mâles (26, 28, 30, 32) pouvant être mis en prise hermétiquement avec une pluralité d'éléments formant emboîtements de raccordement de tuyau femelles (152, 154, 156, 158) dans la portion de raccord femelle ; les éléments formant bouts unis et les éléments formant emboîtements possédant des moyens de réception de tuyau (46, 48, 50, 52) ; des moyens de mise en prise mutuelle (120) dans les portions de raccord pour maintenir les portions de raccord mâle et femelle ensemble ou pour permettre la séparation des portions de raccord, **caractérisé en ce que** le raccord comprend en outre un élément de collier de serrage de tuyau (38) dans chacune des portions de raccord mâle et femelle ; les moyens de mise en prise étant sensibles à des positions endiguant l'écoulement de fluide et permettant l'écoulement de fluide des éléments de collier de serrage (38).

2. Un raccord selon la revendication 1 dans lequel les moyens de réception de tuyau sont choisis parmi : soit des poches destinées à recevoir un tuyau dans celles-ci ; soit des bouts unis tubulaires destinés à recevoir un tuyau sur ceux-ci.

3. Un raccord selon la revendication 1 ou la revendication 2 dans lequel les tuyaux sont de différentes tailles.

4. Un raccord selon une quelconque revendication précédente dans lequel il se trouve, entre les bouts unis mâles et les emboîtements femelles, un moyen d'étanchéité (62, 64).

5. Un raccord selon une quelconque revendication précédente dans lequel les éléments formant bouts unis mâles ou les éléments formant emboîtements femelles sont prévus sur un élément formant collecteur (150).

6. Un raccord selon la revendication 5 dans lequel les éléments de l'autre groupe d'entre les éléments formant bouts unis mâles ou les éléments formant emboîtements femelles sont individuellement situés à l'intérieur de la portion de raccord.

7. Un raccord selon une quelconque revendication précédente dans lequel l'élément de collier de serrage de tuyau (38) possède des orifices (74, 76, 78, 80) au travers desquels passent des tuyaux dans un état d'écoulement de fluide et des portions en fentes (66, 68, 70, 72) dans lesquelles des tuyaux sont fermés par compression dans un état de non-écoulement de fluide.

8. Un raccord selon la revendication 7 dans lequel dans l'élément de collier de serrage de tuyau (38), les portions en fentes (66, 68, 70, 72) sont raccordées aux orifices au niveau d'une périphérie de ceux-ci.

9. Un raccord selon la revendication 7 ou la revendication 8 dans lequel l'élément de collier de serrage de tuyau (38) est déplaçable dans une direction substantiellement parallèle auxdites portions en fentes.

10. Un raccord selon l'une quelconque des revendications précédentes 7 à 9 dans lequel tous les tuyaux sont ouverts ou fermés simultanément grâce au déplacement de l'élément de collier de serrage de tuyau (38).

11. Un raccord selon une quelconque revendication précédente dans lequel l'élément de collier de serrage de tuyau (38) est déplaçable dans une direction transversale aux axes des éléments de raccordement formant bouts unis et emboîtements.

12. Un raccord selon une quelconque revendication précédente dans lequel les moyens de mise en prise (120) sont maintenus en prise pour maintenir les portions de raccord mâle et femelle ensemble quand l'élément de collier de serrage de tuyau se trouve dans une position permettant l'écoulement de fluide.

13. Un raccord selon une quelconque revendication précédente dans lequel les moyens de mise en prise mutuelle (120) peuvent être libérés quand les éléments de collier de serrage de tuyau se trouvent dans une position endiguant l'écoulement de fluide.

14. Un raccord selon une quelconque revendication précédente dans lequel les moyens de mise en prise mutuelle (120) comprennent des portions de mise en prise en doigts (128, 134) et en fentes (129, 136).

15. Un raccord selon la revendication 14 dans lequel l'élément de collier de serrage de tuyau (38) possède un moyen formant came (122, 125) qui pousse les portions en doigts et en fentes à se mettre en prise lorsque l'élément de collier de serrage de tuyau se trouve dans une position permettant l'écoulement de fluide.

16. Un raccord selon une quelconque revendication précédente dans lequel les portions mâle et femelle (10, 12) sont façonnées afin d'empêcher un assemblage incorrect.
